# EUROPÄISCHE PATENTSCHRIFT

(11) **EP 4 061 293 B1**
(45) Veröffentlichungstag und Bekanntmachung des Hinweises auf die Patenterteilung: **11.10.2023**
(21) Anmeldenummer: 20800811.0
(22) Anmeldetag: 27.10.2020
(51) Int. Cl.: A61F 5/01, A43B 17/00, A43D 1/02, A61F 5/14, B29D 35/12, B29D 35/14, G06T 17/00

(54) **VERFAHREN ZUM ERSTELLEN EINES 3 D-SCANS**
METHOD FOR CREATING A 3D SCAN
PROCÉDÉ POUR RÉALISER UNE NUMÉRISATION 3D

(30) Priorität: 19.11.2019 DE 102019131234
(43) Veröffentlichungstag der Anmeldung: 28.09.2022
(73) Patentinhaber: Ottobock SE & Co. KGaA, 37115 Duderstadt (DE)
(72) Erfinder: VOLKMAR, Julia, 37115 Duderstadt (DE); WILLE, Judith, 37308 Heilbad Heiligenstadt (DE)
(74) Vertreter: Gramm, Lins & Partner Patent- und Rechtsanwälte PartGmbB
(86) Internationale Anmeldenummer: PCT/EP2020/080097
(87) Internationale Veröffentlichungsnummer: WO 2021/099069

(56) Entgegenhaltungen:
- WO-A1-2012/052044
- DE-A1-102013 002 012
- DE-A1-102014 102 628

## Beschreibung

Die Erfindung betrifft ein Verfahren zum Erstellen eines 3 D-Scans eines Körperteils wobei Anlageelement in einem derartigen Verfahren verwendet wird. Die Erfindung wird in den Ansprüchen definiert.

In unterschiedlichen Bereichen der Orthopädietechnik ist es notwendig, Gegenstände oder Bauteile von Gegenständen individuell an Körperteile eines Patienten anzupassen. Dies betrifft sowohl Orthesen als auch Prothesen, Prothesenschäfte und andere Unterstützungsvorrichtungen, die beispielsweise bei schweren körperlichen Arbeiten unterstützen sollen. Aus dem Stand der Technik sind unterschiedliche Verfahren bekannt, den Körperteil, der durch eine orthopädietechnische Einrichtung versorgt werden soll, abzuformen oder zu scannen, um eine Negativform und/oder eine positive Form des Körperteils zu erhalten oder herstellen zu können. Aus der DE 10 2013 002 012 A1 ist beispielsweise ein Verfahren bekannt, bei dem ein Fuß einen Abdruck in einem Schaumblock hinterlässt, der anschließend gescannt wird. Die WO 2012/052044 A1 lehrt hingegen, direkt einen Fuß mit einem Fuß-Scanner zu erfassen. Die jeweils erhaltenen Daten werden mit 2-dimensionalen Daten des Fußes kombiniert und zum Herstellen einer orthopädischen Einlage verwendet.

Trotz der großen Fortschritte, die die Scanner-Technologie und die Scan-Technik in den letzten Jahren gemacht hat, wird in der Orthopädietechnik häufig der zu versorgende Körperteil eingegipst, um nach dem Aushärten des Gipses eine Negativform des jeweiligen Körperteils zu erhalten. Der Vorteil dieses Verfahrens besteht darin, dass ein Orthopädietechniker, der das Verfahren durchführt, in Kontakt mit dem Körperteil kommt und so in der Lage ist, eine Negativform des Körperteils unter einer bestimmten Belastung oder Formgebung zu erhalten. So ist es beispielsweise von Vorteil, einen Amputationsstumpf, beispielsweise einen Oberschenkel-Amputationsstumpf, beim Abformen mit dem Gips zu palpieren, also insbesondere tastend zu untersuchen. Auf diese Weise können beispielsweise die Lage, die Position und die Länge von Knochen, Weichteilen oder Narbengewebe bestimmt und in die Formgebung einbezogen werden. Insbesondere erfahrener Orthopädietechniker wollen auf diese haptische Rückmeldung, die sie auf diese Weise erhalten, nicht verzichten, da bei nachträglichen Anpassungen an einem Computer-Modell diese Rückmeldung nicht vorhanden ist.

Diese Art der Formgebung ist auch bei der Herstellung von Orthesen von Vorteil. Soll beispielsweise eine Fußheberorthese hergestellt werden, die verwendet wird, um einen Fuß unterstützend anzuheben, ist es wenig sinnvoll, den Fuß im entlasteten Zustand abzuformen, da der Patient gerade nicht in der Lage ist, den Fuß selbst anzuheben. Vielmehr ist es für die Orthese sinnvoll und wichtig, den Fuß im angehobenen Zustand, der durch die Orthese unterstützt oder möglich gemacht werden soll, abzuformen. Auch dies geht nur, wenn der Orthopädietechniker in Kontakt mit dem abzuformenden Körperteil, vorliegend also dem Fuß, ist.

Diese haptische Interaktion mit dem abzuformenden Körperteil, die für ein möglichst gutes Ergebnis notwendig und wichtig ist, steht der Verwendung von berührungslose Scan-Technik entgegen. Diese verfügt jedoch über eine Reihe anderer Vorteile. Wird ein Körperteil des Trägers eingegipst, ist dies für den Träger unangenehm, unbequem und mit einem relativ hohen Zeitaufwand verbunden. Zudem wird der Körperteil stark verschmutzt. Soll der hergestellte Gips später vom Körperteil abgenommen werden, muss er zudem aufgeschnitten oder zersägt werden, um den Körperteil aus dem Gips entfernen zu können. Dabei kann es zu Beschädigungen der Form kommen, die ausgeglichen und korrigiert werden müssen.

Der Erfindung liegt daher die Aufgabe zugrunde, ein Verfahren zum Erstellen eines 3 D-Scans eines Körperteiles so zu verbessern, dass die Nachteile aus dem Stand der Technik behoben oder zumindest vermindert werden.

Die Erfindung löst die gestellte Aufgabe durch ein Verfahren zum Erstellen eines 3 D-Scans eines Körperteils, wobei das Verfahren folgende Schritte aufweist: a) Anformen eines Anlageelementes an dem Körperteil, b) Erfassen eines ersten Teilscans eines Körperteils, wobei ein Teil des Körperteils von dem Anlageelement überdeckt ist, c) Entfernen des Körperteils von dem Anlageelement, d) Erfassen eines zweiten Teilscans der Fläche des Anlageelements, mit der das Anlageelement an dem Körperteil angelegt war, und e) Erstellen des 3 D-Modells zumindest auch aus dem ersten Teilscan und dem zweiten Teilscan.

Mit dem erfindungsgemäßen Verfahren werden die Vorteile der aus dem Stand der Technik bekannten Verfahren kombiniert. Zunächst wird ein Anlageelement an dem Körperteil angeformt. Dabei kann der Orthopädietechniker, der das Verfahren durchführt, haptische, beispielsweise palpierend, tastend oder korrigierend auf den abzuformenden Körperteil einwirken. Dies kann in einem unbelasteten oder in einem belasteten Zustand des Körperteils geschehen. Das Anlageelement ist bevorzugt verformbar ausgebildet und kann auf diese Weise in die gewünschte Form gebracht werden, wobei besonders bevorzugt gleichzeitig der Körperteil in die gewünschte Form gebracht werden kann. Nachdem dies geschehen ist, wird zunächst nur ein erster Teilscan des Körperteils erfasst. Dazu können aus dem Stand der Technik bekannte 3 D-Scanner verwendet werden. Besonders einfach ist die Handhabung mit einem mobilen Hand-Scanner, also einem Scanner, der in der Hand gehalten und bewegt werden kann. Auf diese Weise kann der Scanner besonders einfach derart um den Körperteil herum bewegt werden, dass der erste Teilscan das Körperteil möglichst vollständig erfasst.

Für einen vollständigen 3 D-Scan des Körperteils, aus dem später ein 3D-Modell erstellt werden kann, ist es jedoch nötig, auch die Fläche des Körperteils zu erfassen, die von dem Anlageelement überdeckt und daher für den Scanner nicht sichtbar ist. Um dies zu erreichen wird der Körperteil vom Anlageelement entfernt. Dann wird die Fläche, mit der das Anlageelement an dem Körperteil angelegt war, in Form eines zweiten Teilscans erfasst. Die in diesem Fall sichtbare und gescannte Oberfläche des Anlageelementes ist eine Negativform der Kontaktfläche des Körperteils mit den vom Orthopädietechniker oder einem anderen Benutzer des Verfahrens vorgenommenen Änderungen und/oder Anpassungen. Auf diese Weise ist der sichtbare Teil des Körperteils im ersten Teilscan erfassbar, während der unsichtbare Teil des Körperteils, nämlich der Teil, der von dem Anlageelement überdeckt war, im zweiten Teilscan als Negativform des Anlageelementes erfasst wird. Anschließend können die beiden Teilscans in einer elektronischen Datenverarbeitungseinrichtung zusammengeführt werden, sodass ein bevorzugt vollständiger 3 D-Scans des Körperteils und daraus ein 3D-Modell erstellt werden kann.

Mit diesem Verfahren ist es für die Person, die das Verfahren durchführt, insbesondere dem Techniker, möglich, die haptische Rückmeldungen und Eindrücke, die das Gips-Verfahren auch heute noch so vorteilhaft machen, zu erhalten und zu bekommen ohne gleichzeitig die hohe Verschmutzung, den hohen Zeitaufwand und den hohen manuellen Aufwand des Gips-Verfahrens zu haben. Gleichzeitig können die sehr guten und genauen Scans, die durch einen 3-D-Scanner erhalten werden können, verwendet werden. Insbesondere für den Fall, dass auf der Grundlage des so erstellten 3 D-Scans ein Bauteil maschinell erzeugt werden soll, beispielsweise mittels eines 3-D-Druckers, einer CNC-Fräse oder eines sonstigen computergesteuerten Werkzeugs, ist dieses Vorgehen von Vorteil, da die elektronisch vorhandenen Daten des 3 D-Scans, der in der elektronischen Datenverarbeitungseinrichtung erstellt wird, mit oder ohne zusätzliche Bearbeitung direkt dem jeweiligen Werkzeug zur Verfügung gestellt werden können.

Um die wenigstens zwei Teilscans zusammenführen zu können ist es von Vorteil, wenigstens einen Referenzpunkt zu verwenden, der in beiden Teilscans enthalten ist. Zum Erstellen des 3D-Scans zumindest auch aus den wenigsten zwei Teilscans wird der wenigstens eine Referenzpunkt in beiden Teilscans zur Überdeckung gebracht. Bevorzugt sind wenigstens zwei, besonders bevorzugt wenigstens drei Referenzpunkte vorhanden.

Der wenigstens eine Referenzpunkt ist bevorzugt an und/oder auf und/oder in dem Anlageelement angeordnet und wird bei den wenigstens zwei Teilscans mitgescant. Er kann aufgedruckt, aufgeklebt oder in sonstiger Weise mit dem Anlageelement verbunden sein.

Bevorzugt wird das Körperteil vor dem Erfassen des ersten Teilscans derart auf einer Auflagefläche positioniert, dass das Anlageelement zwischen dem Körperteil und der Auflagefläche angeordnet ist. Die Kontaktfläche des Körperteils, mit der er auf der Auflagefläche und damit auf dem zwischen dem Körperteil und der Auflagefläche angeordneten Anlageelement aufliegt, ist dabei in der Regel im ersten Teilscan nicht erfassbar, da sie von außen nicht sichtbar ist. Sie wird im zweiten Teilscan erfasst. Die Auflagefläche dient bevorzugt als Referenzfläche, in der die Referenzpunkte definiert sind. Daher ist es von Vorteil, wenn das Anlageelement nicht relativ zu der Auflagefläche bewegt wird, wenn der Körperteil von dem Anlageelement entfernt wird.

Sinnvoll ist die Verwendung einer Auflagefläche insbesondere für Körperteile, die bei späterer Verwendung des orthopädietechnischen Produktes belastet werden. Die Belastung kann Einfluss auf die äußere Form des Körperteiles, beispielsweise eines Fußes oder eines Beines haben, so dass insbesondere in diesen Fällen, beispielsweise zum Herstellen einer Orthese für den Fuß- und/oder Knöchelbereich, zumindest der erste Teilscan unter Belastung erfasst werden sollte. Ist ein Erfassen eines Teilscans unter Belastung nicht erforderlich, beispielsweise bei der Herstellung mancher Einrichtungen für die obere Extremität, ist eine Durchführung des Verfahrens ohne Auflagefläche vorteilhaft. Soll hier der Stumpf erfasst werden, so kann die Verwendung eines anformbaren Anlageelementes beispielsweise im Bereich des Ellenbogens Vorteile bieten, da hier der Orthopädietechniker regelmäßig Änderungen vornimmt.

In einer bevorzugten Ausgestaltung wird das Anlageelement vor dem Anformen in einen verformbaren Zustand gebracht. Nach dem Anformen an dem Körperteil wird es in einen formstabilen Zustand gebracht. Bevor das Verfahren durchgeführt wird, liegt das Anlageelement bevorzugt im formstabilen Zustand vor. Aus diesem kann es besonders vorzugsweise durch Erwärmen, insbesondere in einem Wasserbad, in einen verformbaren Zustand gebracht werden. Dazu ist das Anlageelement bevorzugt aus einem Kunststoff hergestellt, der thermoplastische Eigenschaften aufweist, also durch Erwärmen in einen verformbaren Zustand bringbar ist. Besonders bevorzugt handelt es sich dabei um ein Material, das bereits bei niedrigen Temperaturen, bevorzugt kleiner als 60° C, besonders bevorzugt kleiner als 55°C, verformbar wird, sodass es auch in diesem Zustand an den Körperteil angelegt werden kann, ohne als unangenehm empfunden zu werden. In diesem Zustand wird das Anlageelement an dem Körperteil angeformt, wie dies bereits beschrieben wurde. Bevorzugt wird das Anlageelement an dem Körperteil festgehalten oder fixiert, bis das Anlageelement wieder in einen formstabilen Zustand überführt wurde. Dies geschieht bevorzugt durch Abkühlen. Dazu ist es oftmals ausreichend, einige Minuten zu warten und das Anlageelement durch thermischen Kontakt mit der Umgebungsluft abzukühlen. Alternativ dazu kann auch ein aktives Abkühlen verwendet werden, bei dem beispielsweise Kühlelemente mit dem Anlageelement in thermischen Kontakt gebracht werden.

Vorzugsweise wird das Anlageelement zwischen dem Erfassen des ersten Teilscans und dem Erfassen des zweiten Teilscans relativ zu einer Referenzmarkierung, die bevorzugt an oder auf der Auflagefläche angeordnet ist, nicht bewegt. Beim Erstellen des 3 D-Scans müssen die Daten des ersten Teilscans und die Daten des zweiten Teilscans in einer elektronischen Datenverarbeitungseinrichtung zusammengeführt werden. Die Daten können dabei zunächst zu einer reinen Kombination der beiden Teilscans zusammengesetzt werden. Alternativ dazu wird direkt ein 3 D-Modell aus der Kombination beider Teilscans erzeugt. Dabei werden bevorzugt weitere Anpassungen vorgenommen und ein Volumenmodell erzeugt. Zum Zusammensetzen der Teilscans ist es von Vorteil, dass die Orientierung und Position der beiden Teilscans und der jeweiligen Daten, die den jeweiligen Teilscan bilden, relativ zueinander bekannt sind. Auf diese Weise können die beiden Teilscans besonders einfach in der richtigen Position und Orientierung zusammengeführt werden. Dazu wird eine Referenzmarkierung verwendet, die sowohl in den Daten des ersten Teilscans als auch in den Daten des zweiten Teilscans identifizierbar ist. Die Referenzmarkierung ist dabei so ausgebildet, dass sie Position und Orientierung der beiden Bilder, also der Teilscans, zueinander eindeutig festlegt. Dazu ist es jedoch von Vorteil und wichtig, dass das Anlageelement zwischen dem Erfassen der beiden Teilscans nicht relativ zur Referenzmarkierung bewegt wird. In einer besonders einfachen Ausgestaltung ist die Auflagefläche Teil einer separaten Vorrichtung, beispielsweise einer Platte, eines Brettes oder einer anderen Unterlage, wobei die Auflagefläche selbst die Referenzmarkierung trägt. Alternativ oder zusätzlich dazu können auch Referenzmarkierungen an dem Körperteil und/oder dem Anlageelement angebracht sein, die das Zusammenfügen von erstem und zweitem Teilscan erleichtern.

Vorzugsweise werden der erste Teilscan und/oder der zweite Teilscan mittels eines 3D-Scanners, bevorzugt eines mobilen 3 D-Scanners erfasst. Wird ein mobiler 3 D-Scanner verwendet, kann dieser relativ zum jeweils zu erfassenden Motiv, also dem Körperteil oder dem Anlageelement, besonders einfach bewegt werden, sodass ein möglichst vollständiger Teilscan insbesondere des Körperteils möglich ist. Wird hingegen ein fest montierter 3 D-Scanner verwendet, ist es möglich, auf eine Referenzmarkierung zu verzichten, sofern das Anlageelement zwischen dem Erfassen des ersten Teilscans und dem Erfassen des zweiten Teilscans relativ zum montierten 3 D-Scanner unbewegt bleibt. Ist in diesem Fall die Orientierung und Position des fest montierten 3 D-Scanners relativ zur Auflagefläche bekannt, kann auf eine zusätzliche separate Referenzmarkierung verzichtet werden.

Die Erfindung löst die gestellte Aufgabe zudem durch ein Anlageelement, das in einem der hier beschriebenen Verfahren verwendbar ist. Das Anlageelement verfügt über eine Ursprungsform und ist, insbesondere durch erwärmen, in einen verformbaren Zustand und, insbesondere durch Abkühlen, in einen formstabilen Zustand bringbar.

In einer bevorzugten Ausgestaltung ist das Anlageelement zumindest auch aus einem Material hergestellt, das sich ohne äußere Krafteinwirkung in eine Ursprungsform verformt, wenn es in den verformbaren Zustand gebracht wird. Besonders bevorzugt besteht das Anlageelement vollständig aus einem derartigen Material. Ein solches Anlageelement ist auf besonders einfache und komfortable Weise mehrfach verwendbar. Nachdem es bei der Durchführung eines der hier beschriebenen Verfahren an den Körperteil angeformt wurde, kann es in den verformbaren Zustand gebracht werden, indem es beispielsweise erwärmt wird. Ohne eine weitere äußere Krafteinwirkung verformt sich das Material dann in die Ausgangsform oder Ursprungsform, in der es ursprünglich hergestellt wurde. Das Material verfügt über ein sogenanntes Formgedächtnis, der auch "Memory"-Effekt genannt wird. Dies bedeutet, dass die einmal hergestellte Ursprungsform auch nach dem Verformen des Anlageelementes beispielsweise in einem hier beschriebenen Verfahren wieder herstellbar ist, indem das Material und damit das Anlageelement in den verformbaren Zustand gebracht wird. Dazu ist es nicht notwendig, eine weitere Kraft auf das Material oder das Anlageelement auszuüben.

Vorzugsweise ist die Ursprungsform an die Form des Körperteils angepasst. Dabei ist diese Anpassung jedoch nicht notwendigerweise an den individuellen Körperteil angepasst, sondern folgt lediglich einer Standardform für dieses Körperteil. Für eine Fußheberorthese ist es beispielsweise von Vorteil, wenn das Anlageelement derart vorgeformt ist, dass es das Fußgewölbe bereits nachbildet. Auch eine Fersenkappe kann vorzugsweise bereits in der Ursprungsform enthalten sein. Durch diese Anpassung ist es zudem möglich, Stützfunktionen, Kissen oder Polsterungen in das Anlageelement zu integrieren, die dann beim späteren Anformen des Anlageelementes an dem Körperteil nur noch leicht in Position und/oder Orientierung verändert werden müssen. Es hat sich zudem gezeigt, dass ohne eine ausreichende Vorformung der Ursprungsform schnell Falten beim Anformen entstehen, da das Material zu stark verformt werden muss.

Mithilfe der beigefügten Zeichnungen wird nachfolgend ein Ausführungsbeispiel der vorliegenden Erfindung näher erläutert. Es zeigen
- Figuren 1 bis 4: - verschiedene Verfahrensschritte eines Verfahrens gemäß einem Ausführungsbeispiel der vorliegenden Erfindung und
- Figur 5: - ein Anlageelement.

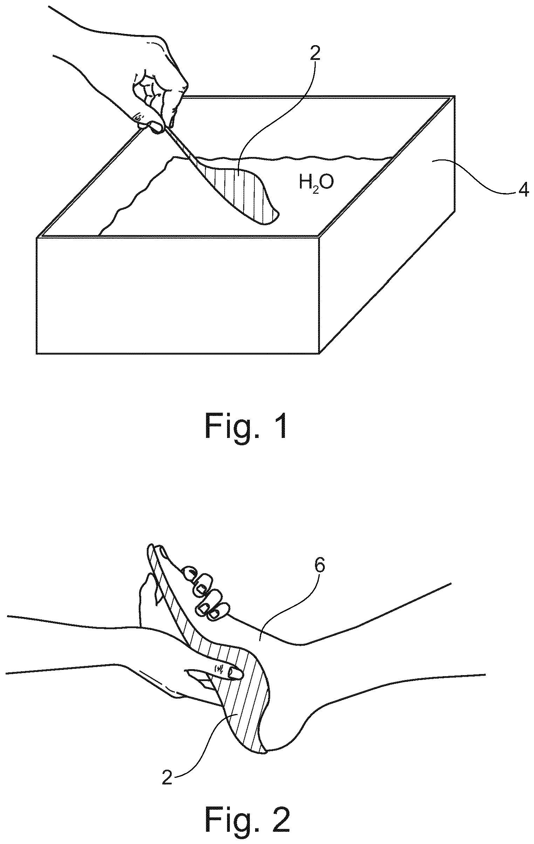

Figur 1 zeigt ein Anlageelement 2, das in einen Behälter 4 eingelegt wird, in dem sich erwärmtes Wasser befindet. Dadurch wird das Anlageelement 2 erwärmt. Es besteht zumindest teilweise, vorzugsweise jedoch vollständig aus einem Material mit einem Formgedächtnis. Ein derartiges Material kann beispielsweise durch Erwärmen in seine Ursprungsform zurückgebracht werden. Dies geschieht im in Figur 1 gezeigten Verfahrensschritt.

Figur 2 zeigt das Anformen des Anlageelementes 2 an den Körperteil 6, der im gezeigten Ausführungsbeispiel ein Fuß ist. Dabei kann, wie in Figur 2 dargestellt, manuell auf die Form des Anlageelementes 2 eingewirkt werden, um es in die gewünschte Form zu drücken. Es wird folglich nicht eine unbelastete Form des Körperteils 6 durch das Anlageelement 2 erfasst, sondern die Person, die das Verfahren durchführt, beispielsweise ein Orthopädietechniker, kann in gewohnter Weise palpieren und so den Körperteil 6 in die gewünschte Form und Belastung bringen, diese kann beispielsweise eine bestimmte Unterstützung je nach medizinischer Indikation vorsehen. In der in Figur 2 gezeigten Position wird das Anlageelement 2 so lange gehalten, bis es ausgehärtet ist und sich in seinem formstabilen Zustand befindet.

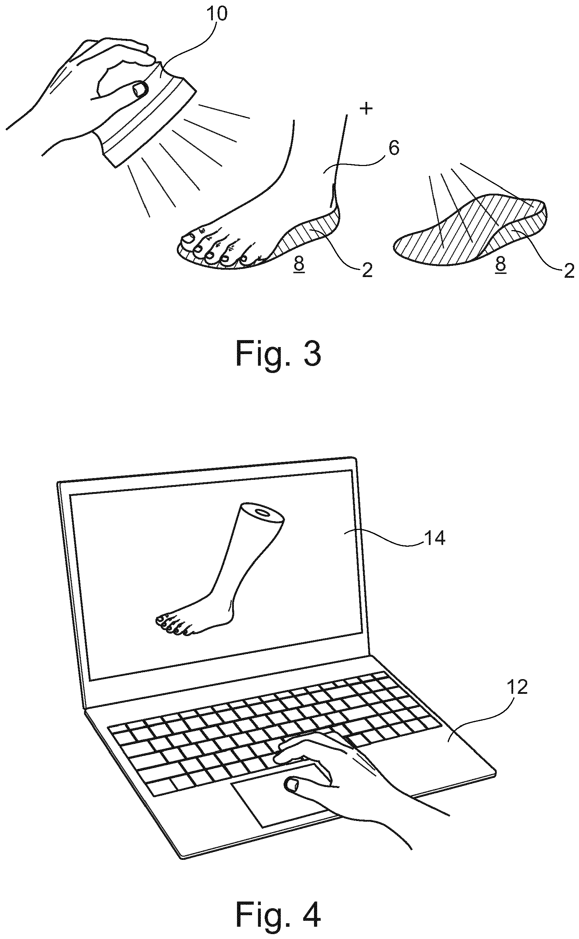

Anschließend wird der Körperteil 6 in diesem Beispiel mit dem Anlageelement 2 auf einer Auflagefläche 8 positioniert, wie dies im linken Teil der Figur 3 dargestellt ist. Mittels eines Scanners 10 wird ein erster Teilscan des Körperteils 6 erfasst. Im gezeigten Ausführungsbeispiel ist der Scanner 10 ein mobiler Scanner, der folglich manuell bewegt und um den Körperteil 6 herum geführt werden kann, sodass sich der erste Teilscan im gezeigten Ausführungsbeispiel um den gesamten Fuß herum erstreckt. Da sich das Anlageelement 2 zwischen der Auflagefläche 8 und dem Körperteil 6 befindet, ist vom Anlageelement 2 nur ein kleiner Teil sichtbar. Nur dieser kleine Teil kann folglich im ersten Teilscan enthalten sein. Daher wird, durch das "+" in Figur 3 angedeutet, ein zweiter Teilscan erfasst, bei dem der Körperteil 6 aus dem Anlageelement 2 entfernt wurde. Dies ist im rechten Teil der Figur 3 dargestellt. Im gezeigten Ausführungsbeispiel befindet sich dabei das Anlageelement 2 weiterhin auf der Auflagefläche 8, die bevorzugt Referenzmarkierungen aufweist. Da sich das Anlageelement 2 in seinem formstabilen Zustand befindet, kann nun durch die Erfassung des Anlageelementes 2 auf den Teil des Körperteils 6 zurückgeschlossen werden, der bei der Erfassung des ersten Teilscans nicht sichtbar war.

Figur 4 zeigt schematisch, dass in einer elektronischen Datenverarbeitungseinrichtung 12 der erste Teilscan und der zweite Teilscan zu einem 3 D-Scan zusammengeführt werden. Auf dem Monitor 14 der elektronischen Datenverarbeitungseinrichtung 12 ist dieser 3-D-Scan dargestellt.

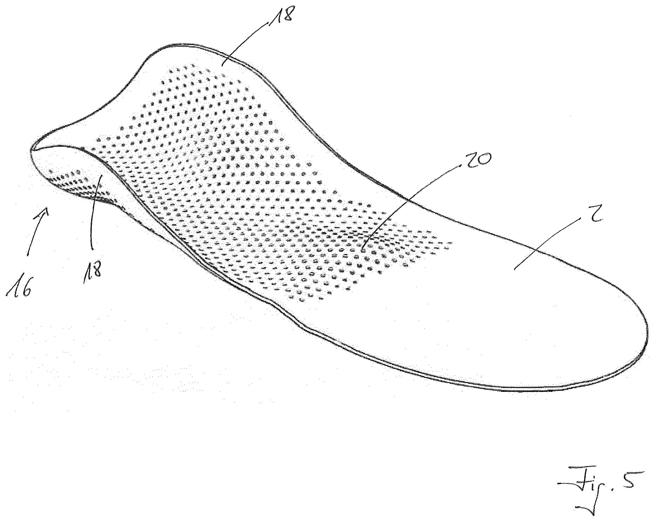

Figur 5 zeigt in einer vergrößerten Darstellung ein Anlageelement 2, das in einem hier beschriebenen Verfahren verwendbar ist. Es befindet sich in Figur 5 in seiner Ursprungsform und ist an die Form einer Fußsohle angepasst. Im Fersenbereich 16 befinden sich 2 nach oben gewölbte Abschnitte 18, während im Bereich der Fußsohle eine polsternde Wulst 20 vorhanden ist, die einem Fußgewölbe nachgebildet ist. Selbst verständlich sind auch andere Formen, Polsterungen, Kissen oder Versteifungen möglich. Diese können dann während des Anformens an den individuellen Körperteil angepasst und herausgearbeitet werden. Die Vorformung bietet dem Orthopädietechniker aber erste Ansatzpunkte, sodass idealerweise nur noch kleinere Anpassungen notwendig sind.

### Bezugszeichenliste

- 2: Anlageelement
- 4: Behälter
- 6: Körperteil
- 8: Auflagefläche
- 10: Scanner
- 12: elektronische Datenverarbeitungseinrichtung
- 14: Monitor
- 16: Fersenbereich
- 18: Abschnitt
- 20: Wulst

## Patentansprüche

1. Verfahren zum Erstellen eines 3D-Scans eines Körperteils, wobei das Verfahren folgende Schritte aufweist:
a. Anformen eines Anlageelementes (2) an den Körperteil (6),
b. Erfassen eines ersten Teilscans des Körperteils, wobei ein Teil des Körperteils von dem Anlageelement überdeckt ist,
c. Entfernen des Körperteils von dem Anlageelement,
d. Erfassen eines zweiten Teilscans der Fläche des Anlageelements, mit der das Anlageelement an dem Körperteil angelegt war, und
e. Erstellen des 3D-Scans zumindest auch aus dem ersten Teilscan und dem zweiten Teilscan.

2. Verfahren nach Anspruch 1, **dadurch gekennzeichnet, dass** das Körperteil vor
dem Erfassen des ersten Teilscans derart auf einer Auflagefläche (8) positioniert
wird, dass das Anlageelement zwischen dem Körperteil und der Auflagefläche angeordnet ist.

3. Verfahren nach Anspruch 1 oder 2, **dadurch gekennzeichnet, dass** das Anlageelement vor dem Anformen in einen verformbaren Zustand gebracht wird, und nach dem Anformen an dem Körperteil in einen formstabilen Zustand gebracht wird.

4. Verfahren nach Anspruch 3, **dadurch gekennzeichnet, dass** das Anlageelement durch Erwärmen, insbesondere in einem Wasserbad, in den verformbaren Zustand gebracht wird.

5. Verfahren nach einem der vorstehenden Ansprüche, **dadurch gekennzeichnet, dass** das Anlageelement zwischen dem Erfassen des ersten Teilscans und dem Erfassen des zweiten Teilscans relativ zu einer Referenzmarkierung, die bevorzugt an oder auf der Auflagefläche angeordnet ist, nicht bewegt wird.

6. Verfahren nach einem der vorstehenden Ansprüche, **dadurch gekennzeichnet, dass** der erste Teilscan und/oder der zweite Teilscan mittels eines 3D-Scanners (10), bevorzugt eines mobilen 3D-Scanners erfasst wird.

7. Verfahren nach einem der vorstehenden Ansprüche, **dadurch gekennzeichnet, dass** das Körperteil ein Fuß ist und das Anlageelement an wenigstens einen Teil der Fußsohle, bevorzugt die gesamte Fußsohle, angeformt wird.

## Claims

1. A method for creating a 3D scan of a body part, the method comprising the following steps:
a. molding a contact element (2) to the body part (6),
b. capturing a first partial scan of the body part, wherein a part of the body part is covered by the contact element,
c. removing the body part from the contact element,
d. capturing a second partial scan of the surface of the contact element with which the contact element was applied to the body part, and
e. creating the 3D scan at least also from the first partial scan and the second partial scan.

2. The method according to claim 1, **characterized in that**, prior to capturing the first partial scan, the body part is positioned on a contact surface (8) in such a way that the contact element is arranged between the body part and the contact surface.

3. The method according to claim 1 or 2, **characterized in that** the contact element is brought into a deformable state prior to molding and, after molding on the body part, it is brought into a dimensionally stable state.

4. The method according to claim 3, **characterized in that** the contact element is brought into the deformable state by heating, in particular in a water bath.

5. The method according to one of the preceding claims, **characterized in that** the contact element is not moved relative to a reference marking, which is preferably arranged on the contact surface, between the capture of the first partial scan and the capture of the second partial scan.

6. The method according to one of the preceding claims, **characterized in that** the first partial scan and/or the second partial scan is captured with a 3D scanner (10), preferably a mobile 3D scanner.

7. The method according to one of the preceding claims, **characterized in that** the body part is a foot and the contact element is molded to at least one part of the sole of the foot, preferably the entire sole of the foot.

## Revendications

1. Procédé de création d'un scan 3D d'une partie du corps, le procédé comprenant les étapes suivantes consistant à :
a. former un élément d'application (2) sur la partie du corps (6),
b. acquérir un premier scan partiel de la partie du corps, une portion de la partie du corps étant recouverte par l'élément d'application,
c. retirer la partie du corps de l'élément d'application,
d. acquérir un deuxième scan partiel de la surface de l'élément d'application par laquelle l'élément d'application était appliqué contre la partie du corps, et
e. créer le scan 3D au moins également à partir du premier scan partiel et du deuxième scan partiel.

2. Procédé selon la revendication 1,
**caractérisé en ce qu'**avant l'acquisition du premier scan partiel, la partie du corps est positionnée sur une surface d'appui (8) de telle sorte que l'élément d'application est disposé entre la partie du corps et la surface d'appui.

3. Procédé selon la revendication 1 ou 2,
**caractérisé en ce que** l'élément d'application est amené dans un état déformable avant le formage et est amené dans un état indéformable après le formage sur la partie du corps.

4. Procédé selon la revendication 3,
**caractérisé en ce que** l'élément d'application est amené dans l'état déformable par chauffage, en particulier dans un bain d'eau.

5. Procédé selon l'une des revendications précédentes,
**caractérisé en ce que** l'élément d'application n'est pas déplacé par rapport à un repère de référence situé de préférence au niveau de la surface d'application ou sur celle-ci, entre l'acquisition du premier scan partiel et l'acquisition du deuxième scan partiel.

6. Procédé selon l'une des revendications précédentes,
**caractérisé en ce que** le premier scan partiel et/ou le deuxième scan partiel sont acquis au moyen d'un scanner 3D (10), de préférence au moyen d'un scanner 3D mobile.

7. Procédé selon l'une des revendications précédentes,
**caractérisé en ce que** la partie du corps est un pied, et l'élément d'application est formé sur au moins une portion de la plante du pied, de préférence sur la totalité de la plante du pied.
